# EUROPÄISCHE PATENTSCHRIFT

(11) **EP 0 881 894 B1**
(45) Veröffentlichungstag und Bekanntmachung des Hinweises auf die Patenterteilung: **30.10.2002**
(21) Anmeldenummer: 97909329.1
(22) Anmeldetag: 29.09.1997
(51) Int. Cl.: A61K 7/13

(54) **FÄRBEMITTEL ZUR FÄRBUNG VON KERATINISCHEN FASERN**
DYEING AGENT FOR DYEING KERATIN FIBRES
AGENT DE COLORATION DE FIBRES KERATINIQUES

(30) Priorität: 05.11.1996 DE 19645538
(43) Veröffentlichungstag der Anmeldung: 09.12.1998
(73) Patentinhaber: Wella Aktiengesellschaft, 64274 Darmstadt (DE)
(72) Erfinder: BRAUN, Hans-Jürgen, CH-3182 Ueberstorf (CH)
(86) Internationale Anmeldenummer: EP9705335
(87) Internationale Veröffentlichungsnummer: WO98019658

(56) Entgegenhaltungen:
- EP-A- 0 400 330
- EP-A- 0 727 203
- WO-A-88/00823
- DE-A- 3 706 565
- DE-A- 3 834 142
- DE-A- 3 942 294

## Beschreibung

Die vorliegende Erfindung betrifft eine Zusammensetzung zur Färbung von keratinischen Fasern, insbesonders von menschlichen Haaren mit einem Gehalt an 2-(2',5'-Diaminophenyl)-ethanol, Resorcin, 2-Methylresorcin, und einem 2-Amino-6-chlor-4-nitrophenol-derivat gemäß der allgemeinen Formel (1).

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels. Von besonderer Bedeutung sind Haarfärbemittel zur Färbung im Naturtonbereich.

An Oxidationsfarbstoffe, die zur Färbung menschlicher Haare verwendet werden sollen, sind zahlreiche besondere Anforderungen gestellt. Die Farbstoffe müssen in toxikologischer und dermatologischer Hinsicht unbedenklich sowie nicht sensibilisierend sein und Färbungen in der gewünschten Intensität ermöglichen.

Ferner wird für die erzielten Haarfärbungen eine gute Licht-, Dauerwell-, Säure- und Reibeechtheit gefordert. Die Haarfärbungen sollen auch ohne Einwirkung von Licht, Reibung und chemischen Mitteln über einen Zeitraum von mindestens vier bis sechs Wochen stabil bleiben.

Außerdem ist es erforderlich, daß durch Kombination geeigneter Entwickler- und Kupplerkomponenten eine breite Palette verschiedener Farbnuancen erzeugt werden kann.

Ein weiteres Problem besteht in der Praxis bezüglich des unterschiedlichen Aufziehverhaltens der Farbstoffe in Abhängigkeit von der Haarbeschaffenheit, wodurch eine ungleichmäßige Anfärbung der Haare erfolgt. Normalerweise neigen die verwendeten Farbstoffe dazu, auf geschädigte Haarpartien stärker aufzuziehen als auf ungeschädigte Haarpartien. Aus diesem Grunde werden in der Regel die durch übliche Alterungsprozesse und Umwelteinflüsse (zum Beispiel Sonnenlicht, Haarwäsche, Färbe- und Dauerwellbehandlungen) stärker geschädigten Haarspitzen intensiver gefärbt als die weniger geschädigten Haarlängen und der Haaransatz, wodurch ein unnatürliches, ungleichmäßiges und völlig unbefriedigendes Färbeergebnis erhalten wird. Dieses Verhalten macht sich insbesondere bei hellen Nuancen sehr störend bemerkbar.

Es bestand daher die Aufgabe, ein Oxidationshaarfärbemittel zur Verfügung zu stellen, das eine gleichmäßige und farbsatte Färbung der Haare im Naturtonbereich von den Haarspitzen bis zum Haaransatz ermöglicht, und gleichzeitig wenig oder gar nicht mutagen und sensibilisierend sowie physiologisch gut verträglich ist.

In der EP-OS 0 688 558 wird die Kombination von 2-(2',5'-Diaminophenyl)ethanol mit 2-Methylresorcin und Resorcin zur Färbung von Haaren in natürlichen Farbtönen empfohlen. In der DE-OS 39 42 294 werden Oxidationshaarfärbemittel beschrieben, welche eine Kombination von 2-(2',5'-Diamino-phenyl)ethanol, Resorcin, 3-Aminophenol und 2-Amino-6-Chlor-4-nitrophenol enthalten.

Außerdem sind mit dem Mittel gemäß der EP-OS 0 688 558 helle Farbtöne nur sehr schwierig nuancierbar.

Überraschenderweise wurde nunmehr gefunden, daß die beschriebenen Probleme bezüglich des Farbausgleiches zwischen Haaransatz und Haarspitzem bei Verwendung einer Kombination aus 2-(2',5'-Diaminophenyl)-ethanol, Resorcin, 2-Methylresorcin und einem 2-Amino-6-chlor-4-nitrophenol der allgemeinen Formel (1) erfolgreich gelöst werden können.

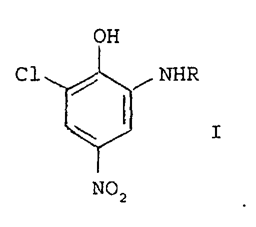

Die beschriebene Farbstoffkombination ermöglicht eine vom Haaransatz bis zur Haarspitze gleichmäßige Färbung im Naturbereich bis zu leicht modischen Färbungen mit rötlichen Reflexen, ganz besonders auch bei helleren Nuancen. Die vorzüglichen Eigenschaften der neuen Farbstoffkombination zeigen sich besonders auf durch Licht und Wetter geschädigtem oder auf dauergewellten Haaren.

Gegenstand der vorliegenden Erfindung ist daher eine Farbträgermasse enthaltend eine Kombination aus 2-(2',5'-Diaminophenyl)-ethanol, Resorcin, 2-Methylresorcin und einem 2-Amino-6-chlor-4-nitrophenol der allgemeinen Formel (1) 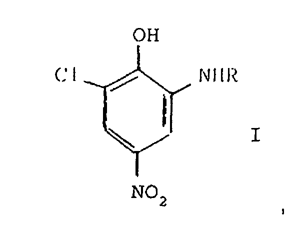 worin R Wasserstoff, eine geradkettige oder verzweigte Alkylgruppe mit 1 bis 6 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet.

Bevorzugte Derivate der allgemeinen Formel (1) sind 2-Amino-6-chlor-4-nitrophenol, 2-Chlor-6-(ethylamino)-4-nitro-phenol und 2-Chlor-6-((2'-hydroxyethyl)amino)-4-nitrophenol.

Zur Abrundung des Farbergebnisses sowie zur Erzeugung von speziellen Farbeffekten können der Farbträgermasse weitere Oxidationsfarb-vorstufen, beispielsweise Derivate des m- oder p-Phenylendiamins, sowie direktziehende Farbstoffe, wie zum Beispiel Azofarbstoffe, Anthrachinone oder Nitrobenzolderivate, zugesetzt werden.

Die vorstehend beschriebene erfindungsgemäße Farbträgermasse wird zur Färbung gemeinsam mit einem Oxidationsmittel in einer geeigneten kosmetischen Zubereitung appliziert.

Der Gegenstand der vorliegenden Anmeldung betrifft daher auch ein Mittel zur oxidativen Färbung von Haaren, welches unmittelbar vor der Anwendung durch Vermischen der erfindungsgemaßen Farbträgermasse mit einem Oxidationsmittel hergestellt wird.

Die erfindungsgemäße Farbträgermasse enthält die vorstehend beschriebene erfindungsgemäße Kombination von 2-(2',5'-Diaminophenyl)ethanol, Resorcin, 2-Methylresorcin und einem 2-Amino-6-chlor-4-nitrophenolderivat der allgemeinen Formel (1), als solche oder in Form der physiologisch verträglichen Salzen, beispielsweise als Hydrochloride, Sulfate oder Tartrate, oder im Fall von Phenolen als Alkaliphenolate.
Das 2-(2',5'-Diaminophenyl)ethanol wird in der Regel in einer Konzentration von 0,01 bis 10 Gewichtsprozent, vorzugsweise in einer Menge von 0,05 bis 5 Gewichtsprozent, bezogen auf das Gesamtgewicht der Zusammensetzung, eingesetzt, während das Resorcin, das 2-Methylresorcin und das 2-Amino-6-Chlor-4-nitrophenolderivat der allgemeinen Formel (1) jeweils in einer Konzentration von 0,01 bis 5 Gewichtsprozent, vorzugsweise in einer Menge von 0,05 bis 3 Gewichtsprozent, bezogen auf das Gesamtgewicht der Zusammensetzung,eingesetzt wird.

Die Gesamtkonzentration an Farbvorstufen beträgt in der Regel 0,1 bis 10 Gewichtsprozent, vorzugsweise 0,2 und 6 Gewichtsprozent.

Darüberhinaus können in der Farbträgermasse weitere übliche kosmetische Zusätze, beispielsweise Antioxidantien wie Ascorbinsäure, Thioglykolsäure oder Natriumsulfit; Parfümöle; Komplexbildner; Netzmittel und Emulgatoren; Verdicker; Pflegestoffe und andere enthalten sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Das gebrauchsfertige erfindungsgemäße Haarfärbemittel wird unmittelbar vor der Anwendung durch Vermischen der erfindungsgemäßen Farbträgermasse mit einem flüssigen Oxidationsmittel hergestellt

Die Farbträgermasse und das Oxidationsmittel werden hierbei im Gewichtsverhältnis von 5:1 bis 1:3 miteinander vermischt, wobei ein Gewichtsverhältnis von 1:1 bis 1:2 besonders bevorzugt ist.

Des pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimenge in der Farbträgermasse und die Säuremenge im Oxidationsmittel sowie durch das Mischungsverhältnis bestimmt wird. Der pH-Wert des gebrauchsfertigen Haarfärbemittels beträgt in der Regel 3 bis 11, vorzugsweise 5 bis 9.

Für die Einstellung des pH-Wertes der Farbträgermasse und des Oxidationsmittels können je nach dem gewünschten pH-Wert organische und anorganische Säuren, wie zum Beispiel Phosphorsäure, Ascorbinsäure oder Milchsäure, oder Alkalien wie zum Beispiel Monoethanolamin, Triethanolamin, 2-Amino-2-methyl-1-propanol, Ammoniak, Natronlauge, Kalilauge oder Tris(hydroxymethyl)-amino-methan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm, des erhaltenen gebrauchsfertigen Oxidationshaarfärbemittels auf das Haar auf.

Als Oxidationsmittel kommen insbesondere Wasserstoffperoxid oder dessen Additionsverbindungen an Harnstoff, Melamin oder Natriumbromat in Form einer 1- bis 12prozentigen, vorzugsweise 6prozentigen, wäßrigen Lösung in Betracht, wobei Wasserstoffperoxid besonders bevorzugt wird.

Man läßt das erfindungsgemäße Haarfärbemittel bei 15 bis 50 Grad Celsius etwa 10 bis 45 Minuten lang, vorzugsweise 30 Minuten lang, auf das Haar einwirken, spült sodann das Haar mit Wasser und trocknet es. Gegebenenfalls wird im Anschluß an diese Spülung mit einem Shampoo gewaschen und/oder mit einer schwachen organischen Säure, wie zum Beispiel Zitronensäure oder Weinsäure, nachgespült. Anschließend wird das Haar getrocknet.

Alle Prozentangaben stellen soweit nicht anders angegeben Gewichtsprozente dar

Die nachfolgenden Beispiele sollen den Gegenstand näher erläutern, ohne ihn auf diese Beispiele zu beschränken.

### Beispiele

### Beispiel 1: Haarfärbelösung

| | |
|---|---|
| 0,67 g | 2-(2',5'-Diaminophenyl)-ethanol-sulfat |
| 0,20 g | Resorcin |
| 0,20 g | 2-Methylresorcin |
| 0,30 g | 2-Amino-6-chlor-4-nitrophenol |
| 10,00 g | Isopropanol |
| 10,00 g | Laurylalkohol-diglykolether-sulfat-Natriumsalz |
| | (28prozentige wäßrige Lösung) |
| 10,00 g | Ammoniak (25prozentige wäßrige Lösung) |
| 0,30 g | Ascorbinsäure |
| 68,33 g | Wasser vollentsalzt |
| 100,00 g | |

Zur Anwendung werden 10 g Haarfärbelösung mit 10 g Wasserstoffperoxid-Lösung (6prozentige wäßrige Lösung) gemischt Das erhaltene Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar hat einen gleichmäßig goldbraunen Farbton angenommen.

### Beispiel 2: Haarfärbecreme

| | |
|---|---|
| 0,3 g | 2-(2,5-Diaminophenyl)-ethanol-sulfat |
| 0,1 g | Resorcin |
| 0,1 g | 2-Methylresorcin |
| 0,2 g | 2-Chlor-6-(ethylamino)-4-nitrophenol |
| 15,0 g | Cetylalkohol |
| 3,5 g | Laurylalkohol-diglykolether-sulfat-Natriumsalz |
| | (28prozentige wäßrige Lösung) |
| 3,0 g | Ammoniak (25prozentige wäßrige Lösung) |
| 0,3 g | Natrimsulfit, wasserfrei |
| 77,5 g | Wasser vollentsalzt |
| 100,0 g | |

Zur Anwendung werden 10 g Haarfärbecreme mit 10 g Wasserstoffperoxid-Lösung (6-prozentige wäßrige Lösung) gemischt. Das erhaltene gebrauchsfertige Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar ist vom Ansatz bis zur Spitze in einem gleichmäßigen Kupfer-Ton gefärbt.

## Patentansprüche

1. Haarfarbträgermasse **dadurch gekennzeichnet, daß** sie eine Kombination aus 2-(2',5'-Diaminophenyl)-ethanol, Resorcin, 2-Methylresorcin und einem 2-Amino-6-Chlor-4-nitrophenol der allgemeinen Formel (I) 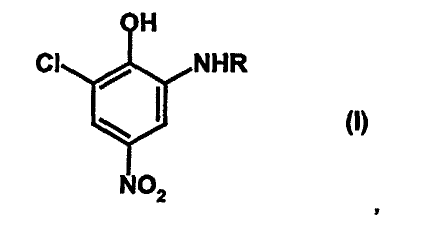 worin R Wasserstoff, eine geradkettige oder verzweigte Alkylgruppe mit 1 bis 6 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet, oder deren physiologisch verträglichen Salzen enthält.

2. Haarfarbträgermasse nach Anspruch 1, **dadurch gekennzeichnet, daß** das 2-Amino-6-chlor-4-nitrophenol der allgemeinen Formel (1) ausgewählt ist 2-Amino-6-chlor-4-nitrophenol, 2-Chlor-6-ethylamino-4-nitrophenol und 2-Chlor-6-((2'-hydroxyethyl)amino)-4-nitrophenol.

3. Haarfarbträgermasse nach Anspruch 1 oder 2, **dadurch gekennzeichnet, daß** das 2-(2',5'-Diaminophenyl)-ethanol in Form seines Additionssalzes mit einer anorganischen oder organischen Säure enthalten ist.

4. Haarfarbträgermasse nach mindestens einem der Ansprüche 1 bis 3, **dadurch gekennzeichnet, daß** das 2-(2',5'-Diaminophenyl)-ethanol wird in einer Menge von 0,01 bis 10 Gewichtsprozent, bezogen auf das Gesamtgewicht der Zusammensetzung, eingesetzt wird.

5. Haarfarbträgermasse nach mindestens einem der Ansprüche 1 bis 3, **dadurch gekennzeichnet, daß** das Resorcin, bezogen auf das Gesamtgewicht der Zusammensetzung, in einer Menge von 0,01 bis 5 Gewichtsprozent eingesetzt wird

6. Haarfarbträgermasse nach mindestens einem der Ansprüche 1 bis 3, **dadurch gekennzeichnet, daß** das 2-Methylresorcin, bezogen auf das Gesamtgewicht der Zusammensetzung, in einer Menge von 0,01 bis 5 Gewichtsprozent eingesetzt wird.

7. Haarfarbträgermasse nach mindestens einem der Ansprüche 1 bis 3, **dadurch gekennzeichnet, daß** das 2-Amino-6-chlor-4-nitrophenolderivat der allgemeinen Formel (1), bezogen auf das Gesamtgewicht der Zusammensetzung, in einer Menge von 0,01 bis 5 Gewichtsprozent eingesetzt wird.

8. Haarfarbträgermasse nach mindestens einem der Ansprüche 1 bis 7, **dadurch gekennzeichnet, daß** die Farbvorstufen, bezogen auf das Gesamtgewicht der Zusammensetzung, in einer Gesamtmenge von 0,1 bis 10 Gewichtsprozent eingesetzt werden.

9. Haarfarbträgermasse nach mindestens einem der Ansprüche 1 bis 8, **dadurch gekennzeichnet, daß** sie übliche kosmetische Zusätze enthält.

10. Haarfärbemittel, **dadurch gekennzeichnet, daß** es unmittelbar vor der Anwendung durch Vermischen einer Haarfarbträgermasse nach mindestens einem der Ansprüche 1 bis 9 mit einem Oxidationsmittel hergestellt wird.

11. Haarfärbemittel nach Anspruch 10, **dadurch gekennzeichnet, daß** die Haarfarbträgermasse mit dem Oxidationsmittel in einem Verhältnis von 5:1 bis 1:3 vermischt wird.

12. Haarfärbemittel nach Anspruch 10 oder 11, **dadurch gekennzeichnet, daß** es einen pH-Wert von 3 bis 11 aufweist.

13. Haarfärbeverfahren, **dadurch gekennzeichnet, daß** ein Haarfärbemittel nach mindestens einem der Ansprüche 10 bis 12 auf das Haar aufgetragen wird, bei einer Temperatur von 15 bis 50°C 10 bis 45 Minuten lang einwirken gelassen wird, das Haar anschließend mit Wasser gespült wird, gegebenenfalls schamponiert und sodann getrocknet wird.

## Claims

1. Hair dye carrier mass **characterized in that** it comprises a combination of 2-(2',5'-diaminophenyl)-ethanol, resorcinol, 2-methylresorcinol and a 2-amino-6-chloro-4-nitrophenol of the general formula (I) 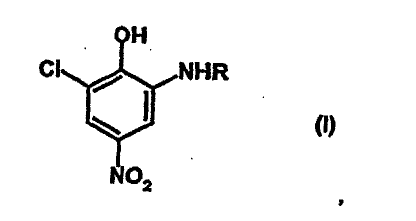 in which R is hydrogen, a straight-chain or branched alkyl group having 1 to 6 carbon atoms, a straight-chain or branched hydroxyalkyl group having 2 to 4 carbon atoms or a straight-chain or branched polyhydroxyalkyl group having 3 to 4 carbon atoms, or physiologically compatible salts thereof.

2. Hair dye carrier mass according to Claim 1, **characterized in that** the 2-amino-6-chloro-4-nitrophenol of the general formula (I) is chosen from 2-amino-6-chloro-4-nitrophenol, 2-chloro-6-ethylamino-4-nitrophenol and 2-chloro-6-((2'-hydroxyethyl)amino)-4-nitrophenol.

3. Hair dye carrier mass according to Claim 1 or 2, **characterized in that** the 2-(2',5'-diaminophenyl)-ethanol is present in the form of its addition salt with an inorganic or organic acid.

4. Hair dye carrier mass according to at least one of Claims 1 to 3, **characterized in that** the 2-(2',5'-diaminophenol)ethanol is used in an amount of from 0.01 to 10 per cent by weight, based on the total weight of the composition.

5. Hair dye carrier mass according to at least one of Claims 1 to 3, **characterized in that** the resorcinol, based on the total weight of the composition, is used in an amount of from 0.01 to 5 per cent by weight.

6. Hair dye carrier mass according to at least one of Claims 1 to 3, **characterized in that** the 2-methylresorcinol, based on the total weight of the composition, is used in an amount of from 0.01 to 5 per cent by weight.

7. Hair dye carrier mass according to at least one of Claims 1 to 3, **characterized in that** the 2-amino-6-chloro-4-nitrophenol derivative of the general formula (I), based on the total weight of the composition, is used in an amount of from 0.01 to 5 per cent by weight.

8. Hair dye carrier mass according to at least one of Claims 1 to 7, **characterized in that** the die precursors, based on the total weight of the composition, are used in a total amount of from 0.1 to 10 per cent by weight.

9. Hair dye carrier mass according to at least one of Claims 1 to 8, **characterized in that** it comprises customary cosmetic additives.

10. Hair dyeing agent **characterized in that** it is prepared directly prior to use by mixing a hair dye carrier mass according to at least one of Claims 1 to 9 with an oxidizing agent.

11. Hair dyeing agent according to Claim 10, **characterized in that** the hair dye carrier mass is mixed with an oxidizing agent in a ratio of from 5:1 to 1:3.

12. Hair dyeing agent according to Claim 10 or 11, **characterized in that** it has a pH of from 3 to 11.

13. Hair dyeing method **characterized in that** a hair dyeing agent according to at least one of Claims 10 to 12 is applied to the hair, left to act at a temperature of from 15 to 50°C for from 10 to 45 minutes, and then the hair is rinsed with water, optionally shampooed and then dried.

## Revendications

1. Matière colorante pour cheveux, **caractérisée en ce qu'**elle contient une association de 2-(2',5'-diaminophényl)éthanol, résorcinol, 2-méthylrésorcinol et d'un 2-amino-6-chloro-4-nitrophénol de formule générale (I) 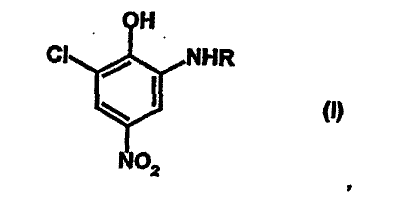 dans laquelle R représente un atome d'hydrogène, un groupe alkyle à chaîne droite ou ramifiée ayant de 1 à 6 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone, ou de leurs sels physiologiquement acceptables.

2. Matière colorante pour cheveux selon la revendication 1, **caractérisée en ce que** le 2-amino-6-chloro-4-nitrophénol de formule générale (I) choisi est le 2-amino-6-chloro-4-nitrophénol, le 2-chloro-6-éthylamino-4-nitrophénol et le 2-chloro-6-((2'-hydroxyéthyl)amino)-4-nitrophénol.

3. Matière colorante pour cheveux selon la revendication 1 ou 2, **caractérisée en ce que** le 2-(2',5'-diaminophényl)éthanol est contenu sous forme de son sel d'addition avec un acide organique ou minéral.

4. Matière colorante pour cheveux selon au moins l'une des revendications 1 à 3, **caractérisée en ce que** le 2-(2',5'-diaminophényl)éthanol est utilisé en une quantité de 0,01 à 10 % en poids, par rapport au poids total de la composition.

5. Matière colorante pour cheveux selon au moins l'une des revendications 1 à 3, **caractérisée en ce que** le résorcinol est utilisé en une quantité de 0,01 à 5 % en poids, par rapport au poids total de la composition.

6. Matière colorante pour cheveux selon au moins l'une des revendications 1 à 3, **caractérisée en ce que** le 2-méthylrésorcinol est utilisé en une quantité de 0,01 à 5 % en poids, par rapport au poids total de la composition.

7. Matière colorante pour cheveux selon au moins l'une des revendications 1 à 3, **caractérisée en ce que** le dérivé de 2-amino-6-chloro-4-nitrophénol de formule générale (I) est utilisé en une quantité de 0,01 à 5 % en poids, par rapport au poids total de la composition.

8. Matière colorante pour cheveux selon au moins l'une des revendications 1 à 7, **caractérisée en ce que** les précurseurs de colorants sont utilisés en une quantité de 0,1 à 10 % en poids, par rapport au poids total de la composition.

9. Matière colorante pour cheveux selon au moins l'une des revendications 1 à 8, **caractérisée en ce qu'**elle contient des additifs cosmétiques usuels.

10. Produit de teinture pour cheveux, **caractérisé en ce qu'**il est préparé immédiatement avant l'emploi, par mélange d'une matière colorante pour cheveux selon au moins l'une des revendications 1 à 9, avec un oxydant.

11. Produit de teinture pour cheveux selon la revendication 10, **caractérisé en ce que** la matière colorante pour cheveux est mélangée avec l'oxydant en un rapport de 5:1 à 1:3.

12. Produit de teinture pour cheveux selon la revendication 10 ou 11, **caractérisé en ce qu'**il présente un pH de 3 à 11.

13. Procédé de teinture des cheveux, **caractérisé en ce qu'**on applique sur les cheveux un produit de teinture pour cheveux selon au moins l'une des revendications 10 à 12, on laisse agir pendant 10 à 45 minutes à une température de 15 à 50°C, puis on rince les cheveux à l'eau, éventuellement on les shampooine et ensuite on les sèche.
